(19) Europäisches Patentamt
European Patent Office
Office européen des brevets

(11) **EP 4 025 906 B1**

(12) **EUROPÄISCHE PATENTSCHRIFT**

(45) Veröffentlichungstag und Bekanntmachung des Hinweises auf die Patenterteilung:
**22.04.2026   Patentblatt 2026/17**

(21) Anmeldenummer: **20767494.6**

(22) Anmeldetag: **01.09.2020**

(51) Internationale Patentklassifikation (IPC):
**G01N 33/00** *(2006.01)*      **G01N 33/497** *(2006.01)*

(52) Gemeinsame Patentklassifikation (CPC):
**G01N 33/497; G01N 33/007; G01N 33/4975**

(86) Internationale Anmeldenummer:
**PCT/EP2020/074295**

(87) Internationale Veröffentlichungsnummer:
**WO 2021/043744 (11.03.2021 Gazette 2021/10)**

(54) **VERFAHREN UND VORRICHTUNG ZUR QUALITÄTSKONTROLLE EINES ATEMMESSGERÄTS UND ATEMMESSGERÄT**

METHOD AND DEVICE FOR QUALITY CONTROL OF A BREATH-MEASURING DEVICE, AND BREATH-MEASURING DEVICE

PROCÉDÉ ET DISPOSITIF POUR LE CONTRÔLE DE QUALITÉ D'UN RESPIROMÈTRE ET RESPIROMÈTRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität:  **03.09.2019   DE 102019213300**

(43) Veröffentlichungstag der Anmeldung:
**13.07.2022   Patentblatt 2022/28**

(73) Patentinhaber: **Robert Bosch GmbH**
**70442 Stuttgart (DE)**

(72) Erfinder:
• THUERSAM, Markus
71263 Weil Der Stadt (DE)
• SCHECK, Kathrin
71334 Waiblingen (DE)
• RIEHL, Sonja
71332 Waiblingen (DE)
• MUELLER, Klaus
70449 Stuttgart (DE)

(56) Entgegenhaltungen:
**US-A1- 2003 216 660     US-B1- 7 063 667**

**Beschreibung**

Stand der Technik

Die Erfindung geht von einer Vorrichtung und einem Verfahren zur Qualitätskontrolle eines Atemmessgeräts und einem Atemmessgerät nach Gattung der unabhängigen Ansprüche aus. Gegenstand der vorliegenden Erfindung ist auch ein Computerprogramm.

Laborgeräte, wie beispielsweise medizinische Geräte, müssen regelmäßig zur Qualitätssicherung auf ihre Funktionsfähigkeit überprüft werden. Die Qualitätssicherung dient dem Nachweis, dass das zu überprüfende Gesamtsystem im angegebenen Mess-, bzw. Analysebereich Ergebnisse innerhalb des zulässigen Genauigkeitsbereichs liefert. Häufig werden zur Durchführung der Qualitätskontrolle bisher Ersatzproben verwendet, die einer menschlichen Probe ähnlich sind.

Die US 2003/21 6660 A1 offenbart ein Atemtestverfahren. Zur Erhöhung der Messgenauigkeit werden Ergebnisse der Atemtests zur kontinuierlichen und automatischen Selbstkalibrierung verwendet.

Offenbarung der Erfindung

Vor diesem Hintergrund werden mit dem hier vorgestellten Ansatz ein verbessertes Verfahren und eine verbesserte Vorrichtung zur Qualitätskontrolle eines Atemmessgeräts und ein verbessertes Atemmessgerät, sowie schließlich ein entsprechendes Computerprogramm gemäß den Hauptansprüchen vorgestellt. Durch die in den abhängigen Ansprüchen aufgeführten Maßnahmen sind vorteilhafte Weiterbildungen und Verbesserungen der im unabhängigen Anspruch angegebenen Vorrichtung möglich.

Durch den hier vorgestellten Ansatz kann bei einem Atemmessgerät eine gleichbleibend hohe Qualität an Messergebnissen auf Basis von menschlichen Proben sichergestellt werden, wodurch eine zuverlässige Therapie von beispielsweise Asthmapatienten ermöglicht wird. In einer ersten Ausführungsform des vorgestellten Ansatzes können menschliche Proben hinsichtlich ihrer Eignung als Referenzwerte überprüft werden. In einer zweiten Ausführungsform können bereits überprüfte menschliche Proben zur Überprüfung des Atemmessgeräts verwendet werden.

Es wird ein Verfahren zur Qualitätskontrolle eines Atemmessgeräts vorgestellt, das ein erstes Teilverfahren zum Qualifizieren einer Testperson und zusätzlich oder alternativ ein zweites Teilverfahren zur Qualitätsprüfung des Atemmessgeräts umfasst. Das Verfahren kann somit als ein Verfahren zum Betreiben des Atemmessgeräts aufgefasst werden, wobei beide Teilverfahren zum erfolgreichen Durchführen der Qualitätskontrolle des Atemmessgeräts beitragen können. Unabhängig davon, ob das erste Teilverfahren oder das zweite Teilverfahren durchgeführt wird, umfasst das Verfahren einen Schritt des Vergleichens und bei Durchführung des ersten Teilverfahrens zusätzlich einen Schritt des Speicherns und bei Durchführung des zweiten Teilverfahrens zusätzlich einen Schritt des Kategorisierens und vorzugsweise ebenfalls einen Schritt des Speicherns von gemessenen Gaskonzentrationen..

Im Schritt des Vergleichens wird ein Prüfwert mit einem Toleranzband verglichen, wobei eine Breite des Toleranzbands durch einen ersten Schwellenwert und einen zweiten Schwellenwert begrenzt wird. Dabei repräsentieren der erste Schwellenwert und der zweite Schwellenwert unter Verwendung eines Mittelwerts und einer Schwankung, beispielsweise einer Standardabweichung, von Referenzwerten sowie einer Genauigkeit des Atemmessgeräts gebildete Werte. Dabei repräsentieren der Prüfwert und die Referenzwerte unter Verwendung des Atemmessgeräts gemessene Gaskonzentrationen.

Im Schritt des Speicherns werden die Referenzwerte für eine nachfolgende Durchführung des zweiten Teilverfahrens gespeichert, wenn der Prüfwert innerhalb des Toleranzbands liegt.

Im Schritt des Kategorisierens wird das Atemmessgeräts als funktionstüchtig oder defekt kategorisiert. Bei einer Kategorisierung als defekt wird dem Nutzer vorzugsweise mitgeteilt, beispielsweise über ein Display angezeigt, dass das Atemmessgerät nicht funktionstüchtig ist und bevorzugt mitgeteilt, dass ein Servicepartner oder Hersteller des Geräts kontaktiert werden muss.

Das Verfahren kann beispielsweise in einem Atemmessgerät durchgeführt werden, das beispielsweise für Personen mit Asthma vorgesehen sein kann. Der Prüfwert kann beispielsweise eine Stickstoffmonoxidmenge anzeigen, die einer menschlichen Atemprobe einer Testperson entnommen wurde, nachdem die Person in das Atemmessgerät hineingepustet hat. Die Atemprobe kann unter Verwendung einer geeigneten Analyseeinrichtung des Atemmessgeräts analysiert werden. Die Analyseeinrichtung kann eine Sensorik umfassen, wie sie bereits im Zusammenhang mit Atemmessgeräten eingesetzt wird. Beispielsweise kann die Analyseeinrichtung ausgebildet sein, um die Stickstoffmonoxidmenge in der Atemprobe zu erfassen und als Messwert abzubilden. Durch den ersten Schwellenwert und den zweiten Schwellenwert kann ein das Toleranzband darstellender Wertebereich begrenzt werden, in dem der Prüfwert angeordnet ist, wenn das Atemmessgerät einsatzbereit ist. Die im ersten Teilverfahren durchgeführte erste Ausführungsform des Verfahrens ermöglicht eine Überprüfung, ob es sich bei den Referenzwerten um Atemproben eines gesunden Menschen handelt. In diesem Fall können die Referenzwerte und der Prüfwert von derselben Person stammen. In der im zweiten Teilverfahren durchgeführten zweiten Ausführungsform des Verfahrens wurden die Referenzwerte bereits überprüft und können nun zur Überprüfung des Atemmessgeräts verwendet werden. Indem die Referenzwerte und der Prüfwert von derselben Person stammen, können Werte einer Person herangezogen und verglichen werden. Bei den Referenzwerten kann es sich um unter Verwendung des Atemmessgeräts erfasste und gespeicherte Messwerte handeln. Die Referenzwerte können beispielsweise aus einer Speichereinrichtung des Atemmessgeräts eingelesen werden. Bei dem Prüfwert kann es sich um einen aktuell unter Verwendung des Atemmessgeräts erfassten Messwert handeln. Der Prüfwert kann beispielsweise aus einer Analyseeinrichtung des Atemmessgeräts eingelesen oder im Falle einer Zwischenspeicherung aus einer Speichereinrichtung des Atemmessgeräts eingelesen werden.

Vorteilhafterweise kann zuerst das erste Teilverfahren zum Qualifizieren einer Testperson und anschließend das zweites Teilverfahren zur Qualitätsprüfung des Atemmessgeräts durchgeführt werden. Beide Teilverfahren können unter Verwendung ein und desselben Atemmessgeräts durchgeführt werden.

Weiterhin umfasst das Verfahren einen Schritt des Bestimmens des Mittelwerts und der Schwankung unter Verwendung der Referenzwerte.

Vorteilhafterweise kann dadurch ein erster Wert mit zumindest einem weiteren Wert, bzw. mit einer Mehrzahl weiterer Werte verglichen werden.

Gemäß einer Ausführungsform kann das Verfahren einen Schritt des Bildens des ersten Schwellenwerts und des zweiten Schwellenwerts unter Verwendung des Mittelwerts und der Schwankung und der Genauigkeit des Atemmessgeräts umfassen. Vorteilhafterweise kann dadurch ein Wertebereich bestimmt werden, in dem sich das Vergleichsergebnis vorteilhafterweise bewegen kann, um beispielsweise eine zuverlässige Therapie von beispielsweise Asthmapatienten sicherzustellen.

Im Schritt des Bildens kann durch den ersten Schwellenwert und den zweiten Schwellenwert eine Bestimmung der Breite des Toleranzbands erfolgen. Dabei können der Mittelwert und die Schwankung in der Bestimmung der Breite antikorrelieren.

Gemäß einer Ausführungsform kann im Schritt des Bildens der Schwellenwerte die Schwankung in Form der Standardabweichung der Referenzwerte mit einem ersten Faktor multipliziert werden, wenn die Standardabweichung oder der Mittelwert kleiner ist als ein vorbestimmter Gaskonzentrationswert. Ferner können die Schwellenwerte mit einem zweiten Faktor multipliziert werden, wenn die Standardabweichung oder der Mittelwert größer ist als der vorbestimmte Gaskonzentrationswert. Vorteilhafterweise kann durch Verwendung des ersten, bzw. des zweiten Faktors eine physiologische Schwankung der Testperson bei einem Funktionalitätstest des Atemgeräts berücksichtigt werden.

Beispielsweise kann im Schritt des Bildens die Schwankung in Form der Standardabweichung der Referenzwerte zum Bilden der Schwellenwerte mit einem von dem Mittelwert abhängigen Faktor multipliziert werden. Dabei können für unterschiedliche Mittelwerte oder unterschiedliche Bereiche von Mittelwerten unterschiedliche Faktoren vorgegeben sein.

Weiterhin kann das Verfahren einen Schritt des Erfassens der Referenzwerte unter Verwendung des Atemmessgeräts umfassen. Somit können Messwerte auf dem Gerät aufgenommen und auch in Form der Referenzwerte dort gespeichert werden.

Gemäß dem ersten Teilverfahren umfasst das Verfahren den Schritt des Speicherns der Referenzwerte für eine nachfolgende Qualitätsprüfung des Atemmessgeräts. Die Referenzwerte können demnach gespeichert werden, wenn die Überprüfung der Referenzwerte ergeben hat, dass es sich um menschliche Proben handelt, die zur Überprüfung des Atemmessgeräts geeignet sind. Dies kann der Fall sein, wenn der Prüfwert größer ist als der erste Schwellenwert und kleiner ist als der zweite Schwellenwert. Optimaler Weise befindet sich der Prüfwert im Wertebereich, sodass die Qualitätsprüfung der Testperson vorteilhafterweise als bestanden klassifiziert wird. Das Speichern ermöglicht eine spätere Nutzung der Referenzwerte zur wiederholten Überprüfung des Atemmessgeräts. Gemäß einer Ausführungsform kann der Prüfwert als ein weiterer Referenzwert gespeichert werden.

Das Verfahren kann ferner einen Schritt des Einlesens des Prüfwerts umfassen. Der Schritt des Einlesens kann nach einer Mindestdauer erneut ausgeführt werden, um zum Veranlassen einer Wiederholung des zweiten Teilverfahrens einen weiteren Prüfwert einzulesen, der eine unter Verwendung des Atemmessgeräts gemessene weitere Gaskonzentration repräsentiert. Das erneute Einlesen kann durchgeführt werden, wenn der Prüfwert kleiner ist als der erste Schwellenwert oder wenn der Prüfwert größer ist als der zweite Schwellenwert, der Prüfwert als außerhalb des Toleranzbereichs liegt. Das bedeutet, dass das Verfahren noch einmal durchgeführt werden kann, wenn das Vergleichsergebnis außerhalb des Wertebereichs liegt. Beispielsweise kann das erste Teilverfahren bei einer erneuten Durchführung mit einer weiteren Testperson im Rahmen einer Qualifizierung durchgeführt werden. In diesem Fall können ferner weitere Referenzwerte der weiteren Testperson eingelesen und anstelle der ursprünglichen Referenzwerte verwendet werden. In dem zweiten Teilverfahren kann das Teilverfahren bei der erneuten Durchführung im Rahmen der Qualitätsprüfung nach beispielsweise einer Wartezeit von etwa 5 Minuten mit derselben Testperson wiederholt werden.

In dem zweiten Teilverfahren kann in einem Schritt des Bereitstellens ein Anzeigesignal bereitgestellt werden, das eine an einen Nutzer gerichtete Frage repräsentiert, wenn nach dem Schritt des erneuten Einlesens der Prüfwert kleiner ist als der erste Schwellenwert oder wenn der Prüfwert größer ist als der zweite Schwellenwert. Das bedeutet, dass beispielsweise nach einem wiederholten Durchlauf des zweiten Teilverfahrens einem Nutzer des Atemmessgeräts die Frage nach einer Erkrankung, beispielsweise einer Erkältung, angezeigt wird, wenn der Prüfwert außerhalb der Schwellenwerte liegt. Vorteilhafterweise kann dadurch eine Fehlfunktion des Atemmessgeräts ausgeschlossen werden, wenn der Nutzer die Frage bestätigt.

Gemäß dem zweiten Teilverfahren kann in einem Schritt des Ausgebens ein Qualitätssignal ausgegeben werden, das eine bestandene Qualitätsprüfung des Atemmessgeräts anzeigt, wenn der Prüfwert größer ist als der erste Schwellenwert und zusätzlich oder alternativ, wenn der Prüfwert kleiner ist als der zweite Schwellenwert. Vorteilhafterweise kann das Atemmessgerät dadurch beispielsweise bei Asthmapatienten eingesetzt werden.

Der Schritt des Einlesens eines weiteren Prüfwerts kann unterbunden werden, wenn der Prüfwert außerhalb des Toleranzbands liegt. Dadurch kann das Atemmessgerät sicherheitshalber gesperrt werden, beispielsweise für eine Mindestzeitdauer.

Dieses Verfahren kann beispielsweise in Software oder Hardware oder in einer Mischform aus Software und Hardware beispielsweise in einem Steuergerät implementiert sein.

Ferner wird ein Atemmessgerät mit einer Vorrichtung vorgestellt, die ausgebildet ist, um das Verfahren gemäß einer der vorangehenden vorgestellten Varianten anzusteuern. Das Atemmessgerät ist dabei ausgebildet, um eine Gaskonzentration zu messen.

Vorteilhafterweise kann dadurch beispielsweise Asthmapatienten geholfen werden, die auf eine Verwendung des Atemmessgeräts angewiesen sind.

Der hier vorgestellte Ansatz schafft ferner eine Vorrichtung, die ausgebildet ist, um die Schritte einer Variante eines hier vorgestellten Verfahrens in entsprechenden Einrichtungen durchzuführen, anzusteuern bzw. umzusetzen. Auch durch diese Ausführungsvariante der Erfindung in Form einer Vorrichtung kann die der Erfindung zugrunde liegende Aufgabe schnell und effizient gelöst werden.

Hierzu kann die Vorrichtung zumindest eine Recheneinheit zum Verarbeiten von Signalen oder Daten, zumindest eine Speichereinheit zum Speichern von Signalen oder Daten, zumindest eine Schnittstelle zu einem Sensor oder einem Aktor zum Einlesen von Sensorsignalen von dem Sensor oder zum Ausgeben von Daten- oder Steuersignalen an den Aktor und/oder zumindest eine Kommunikationsschnittstelle zum Einlesen oder Ausgeben von Daten aufweisen, die in ein Kommunikationsprotokoll eingebettet sind. Die Recheneinheit kann beispielsweise ein Signalprozessor, ein Mikrocontroller oder dergleichen sein, wobei die Speichereinheit ein Flash-Speicher, ein EEPROM oder eine magnetische Speichereinheit sein kann. Die Kommunikationsschnittstelle kann ausgebildet sein, um Daten drahtlos und/oder leitungsgebunden einzulesen oder auszugeben, wobei eine Kommunikationsschnittstelle, die leitungsgebundene Daten einlesen oder ausgeben kann, diese Daten beispielsweise elektrisch oder optisch aus einer entsprechenden Datenübertragungsleitung einlesen oder in eine entsprechende Datenübertragungsleitung ausgeben kann.

Unter einer Vorrichtung kann vorliegend ein elektrisches Gerät verstanden werden, das Sensorsignale verarbeitet und in Abhängigkeit davon Steuer- und/oder Datensignale ausgibt. Die Vorrichtung kann eine Schnittstelle aufweisen, die hard- und/oder softwaremäßig ausgebildet sein kann. Bei einer hardwaremäßigen Ausbildung können die Schnittstellen beispielsweise Teil eines sogenannten System-ASICs sein, der verschiedenste Funktionen der Vorrichtung beinhaltet. Es ist jedoch auch möglich, dass die Schnittstellen eigene, integrierte Schaltkreise sind oder zumindest teilweise aus diskreten Bauelementen bestehen. Bei einer softwaremäßigen Ausbildung können die Schnittstellen Softwaremodule sein, die beispielsweise auf einem Mikrocontroller neben anderen Softwaremodulen vorhanden sind.

Von Vorteil ist auch ein Computerprogrammprodukt oder Computerprogramm mit Programmcode, der auf einem maschinenlesbaren Träger oder Speichermedium wie einem Halbleiterspeicher, einem Festplattenspeicher oder einem optischen Speicher gespeichert sein kann und zur Durchführung, Umsetzung und/oder Ansteuerung der Schritte des Verfahrens nach einer der vorstehend beschriebenen Ausführungsformen verwendet wird, insbesondere wenn das Programmprodukt oder Programm auf einem Computer oder einer Vorrichtung ausgeführt wird.

Ausführungsbeispiele des hier vorgestellten Ansatzes sind in den Zeichnungen dargestellt und in der nachfolgenden Beschreibung näher erläutert. Es zeigt:

Fig. 1 eine schematische Darstellung eines Atemmessgeräts mit einer Vorrichtung gemäß einem Ausführungsbeispiel;

Fig. 2 einen schematischen Ablauf eines Verfahrens zur Qualitätskontrolle eines Atemmessgeräts gemäß einem Ausführungsbeispiel mit zwei Teilverfahren;

Fig. 3 ein Ablaufdiagramm eines Verfahrens zum Qualifizieren einer Testperson gemäß einem Ausführungsbeispiel; und

Fig. 4 ein Ablaufdiagramm eines Verfahrens zur Qualitätsprüfung des Atemmessgeräts gemäß einem Ausführungsbeispiel.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

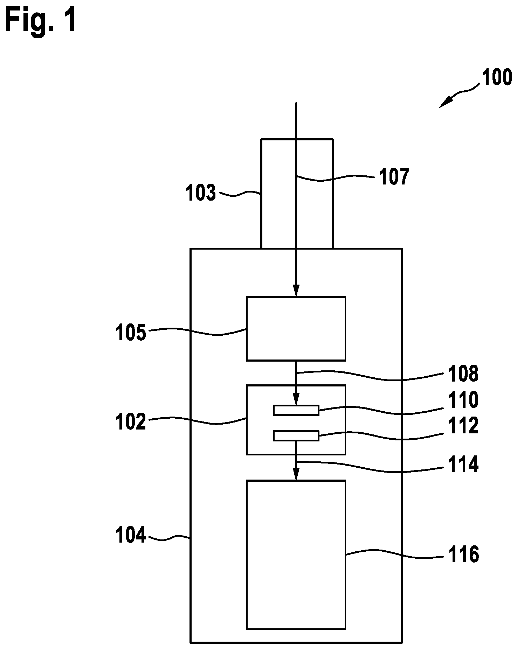

Fig. 1 zeigt eine schematische Darstellung eines Atemmessgeräts 100 mit einer Vorrichtung 102 zur Qualitätskontrolle gemäß einem Ausführungsbeispiel. Das Atemmessgerät 100 ist ausgeformt, um beispielsweise bei einer Therapie von Asthmapatienten eingesetzt zu werden. Bei einem solchen Atemmessgerät 100 ist es unerlässlich, dass eine Qualitätsprüfung durchgeführt wird. Eine solche Qualitätsprüfung kann beispielsweise in regelmäßigen Abständen durchgeführt werden. Dadurch kann eine Funktionalität des Atemmessgeräts 100 kontrolliert werden. Das Atemmessgerät 100 weist gemäß diesem Ausführungsbeispiel ein Mundstück 103, einen Korpus 104, eine Analyseeinrichtung 105 sowie die Vorrichtung 102 zur Qualitätskontrolle auf. Das Mundstück 103 ist ausgebildet, um eine Atemprobe 107 einer Person in den Korpus 104 zu der Analyseeinrichtung 105 zu leiten. Die Analyseeinrichtung 105 ist ausgebildet, um die Atemprobe 107 zu analysieren und einen Messwert bereitzustellen, der ein sich auf die Atemprobe 107 beziehendes Analyseergebnis repräsentiert. Ein solcher Messwert kann als Prüfwert 108 oder Referenzwert eingesetzt werden, wenn unter Verwendung der Vorrichtung 102 die Qualitätskontrolle durchgeführt wird. Der Korpus 104 ist gemäß diesem Ausführungsbeispiel als Gehäuse ausgebildet, um beispielsweise die Vorrichtung 102 und die Analyseeinrichtung 105 vor äußeren Einflüssen zu schützen.

Die Vorrichtung 102 ist ausgebildet, um ein Verfahren zur Qualitätskontrolle des Atemmessgeräts 100 auszuführen, bzw. zu steuern. Dabei ist ein erstes Ausführungsbeispiel des Verfahrens besonders für ein Qualifizieren einer Testperson anwendbar. Wenn sich die Testperson qualifiziert, dann können auf Atemproben der Testperson generierte Messwerte als Referenzwerte verwendet werden. Ein zweites Ausführungsbeispiel des Verfahrens ist besonders bei der Qualitätsprüfung des Atemmessgeräts 100 unter Verwendung solcher Referenzwerte anwendbar.

Bei beiden Ausführungsbeispielen wird ein aktueller Messwert, hier der Prüfwert 108, der eine unter Verwendung der Analyseeinrichtung 105 gemessene Gaskonzentration repräsentiert, von einer Einleseeinheit 110 eingelesen und mit einem ersten Schwellenwert und einem zweiten Schwellenwert unter Verwendung einer Vergleichseinheit 112 verglichen, um ein Vergleichsergebnis 114 zu erhalten. Dabei wird der Prüfwert über eine interne Schnittstelle des Atemmessgeräts 100, beispielsweise von der Analyseeinrichtung 105, eingelesen. Gemäß der Erfindung repräsentieren der erste Schwellenwert und der zweite Schwellenwert unter Verwendung eines Mittelwerts und einer Standardabweichung von Referenzwerten gebildete Werte. Dabei repräsentieren die Referenzwerte ebenfalls unter Verwendung der Analyseeinrichtung 105 gemessene Gaskonzentrationen. Gemäß diesem Ausführungsbeispiel ist das Vergleichsergebnis 114 oder eine unter Verwendung des Vergleichsergebnisses bestimmte Information über eine Anzeigeeinrichtung 116 anzeigbar. Die Anzeigeeinrichtung 116 kann beispielsweise als ein Display realisiert sein. Abhängig von dem Vergleichsergebnis kann unter Verwendung der Anzeigeeinrichtung 116 auch eine Frage oder eine Anweisung an die Testperson ausgegeben werden.

Gemäß dem ersten Ausführungsbeispiel wird unter Verwendung der Anzeigeeinrichtung 116 beispielsweise angezeigt, ob sich eine Person als Testperson eignet oder nicht. Die Person qualifiziert sich als Testperson, wenn sich von der Person bereitgestellte Atemproben 107 zum Bilden von Referenzwerten eignen. In diesem Fall werden die Referenzwerte oder der daraus gebildete Mittelwert und die daraus gebildete Standardabweichung oder die daraus gebildeten Schwellenwerte beispielsweise in einer Speichereinrichtung des Atemmessgeräts 100 gespeichert oder über eine Ausgabeschnittstelle bereitgestellt, um für eine nachfolgende Qualitätsprüfung des Atemmessgeräts 100 verwendet werden zu können. Wenn sich die Person nicht als Testperson qualifiziert, wird beispielsweise unter Verwendung der Anzeigeeinrichtung 116 angezeigt, dass das Verfahren mit einer anderen Person wiederholt werden soll, um geeignete Referenzwerte zu erhalten.

Gemäß dem zweiten Ausführungsbeispiel, bei dem unter Ausführung des ersten Ausführungsbeispiels erhaltene Referenzwerte verwendet werden, wird unter Verwendung der Anzeigeeinrichtung 116 beispielsweise angezeigt, ob das Atemmessgerät 100 die Qualitätsprüfung bestanden hat, oder nicht.

Im Normalbetrieb des Atemmessgeräts 100, also außerhalb der Durchführung einer Qualitätskontrolle, wird unter Verwendung der Anzeigeeinrichtung 116 beispielsweise der von der Analyseeinrichtung 105 aktuell erfasste Messwert oder eine aus dem Messwert bestimmte Information angezeigt. Gemäß einem Ausführungsbeispiel umfasst das Atemmessgerät 100 eine geeignete Steuereinrichtung zum Steuern der von der Anzeigeeinrichtung 116 anzuzeigenden Anzeige.

Mit anderen Worten wird das Atemmessgerät 100 für ein Verfahren zur Qualitätssicherung des Atemmessgeräts 100 zur Atemgasanalyse mit einer menschlichen Probe, hier der Atemprobe 107, unter Berücksichtigung interpersoneller und intrapersoneller physiologischer Schwankungen der Proben vorgestellt. Damit kann gemäß diesem Ausführungsbeispiel eine gleichbleibend hohe Qualität von Messergebnissen sichergestellt werden, wodurch eine zuverlässige Therapie von beispielsweise Asthmapatienten ermöglicht wird. Vorzugsweise wird dazu die Standardabweichung als statisches Maß genutzt, sodass die interpersonellen und intrapersonellen physiologischen Schwankungen berücksichtigt werden.

Im Vergleich zu Asthmatikern oder Personen mit anderen Atemwegserkrankungen zeigen gesunde Personen relativ konstante FeNO-Werte (fraktioniertes exhaliertes Stickstoffmonoxid-Werte). Untersuchungen haben gezeigt, dass eine Person mit niedrigen FeNO-Werten, beispielsweise kleiner als 50ppb, im gesunden Zustand sehr konstante Werte aufzeigt. Bei Werten unter 50ppb liegen die Schwankungen in etwa bei weniger als 3ppb. Ein geeigneter Parameter zur Beobachtung der Konstanz ist daher die Standardabweichung. Bei grundsätzlich gesunden Personen kann es aufgrund der Physiologie zu Schwankungen der FeNO-Werte kommen, die beispielsweise verschiedenen Einflussfaktoren, beispielsweise sportliche Aktivitäten, Treppen steigen oder ein Infekt, zugrunde liegen. Gemäß diesem Ausführungsbeispiel sollte die Standardabweichung idealerweise kleiner als 3 ppb, vorzugsweise kleiner als 1 ppb sein, wenn beispielsweise kein Infekt oder Störungen am Atemmessgerät 100 vorliegt. Steigt die Standardabweichung an, kann gemäß diesem Ausführungsbeispiel darauf geschlossen werden, dass ein Fehler am Atemmessgerät 100 vorliegt oder die Testperson erkrankt ist. Um die physiologischen Schwankungen von einem Defekt, bzw. bei einer Ungenauigkeit des Atemmessgeräts 100 unterscheiden zu können, müssen sie in der Qualitätsprüfung ausgeschlossen werden.

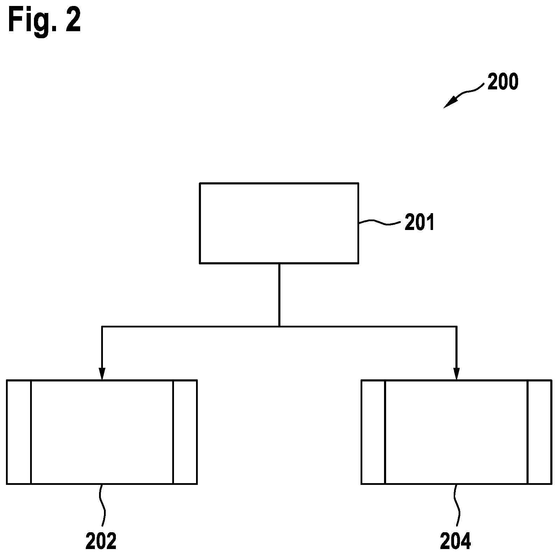

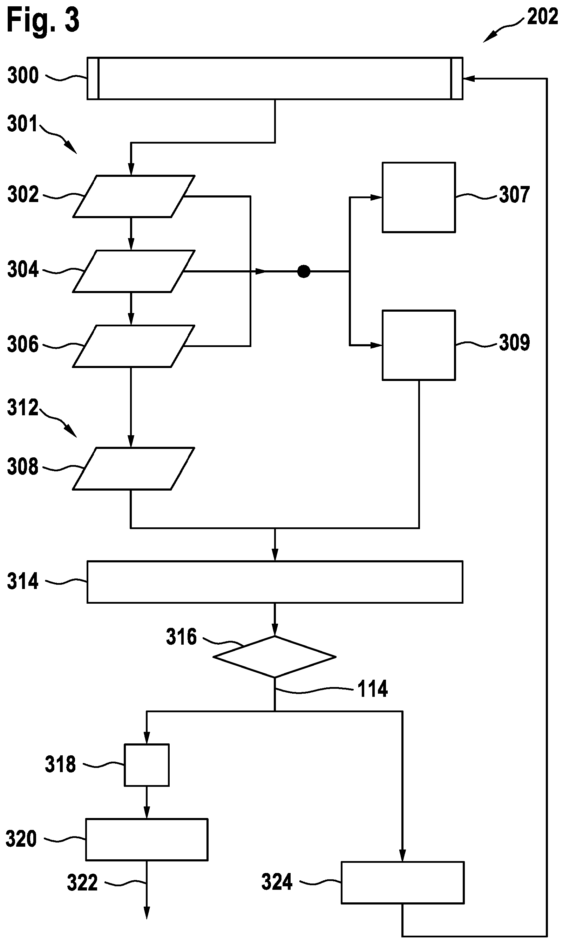

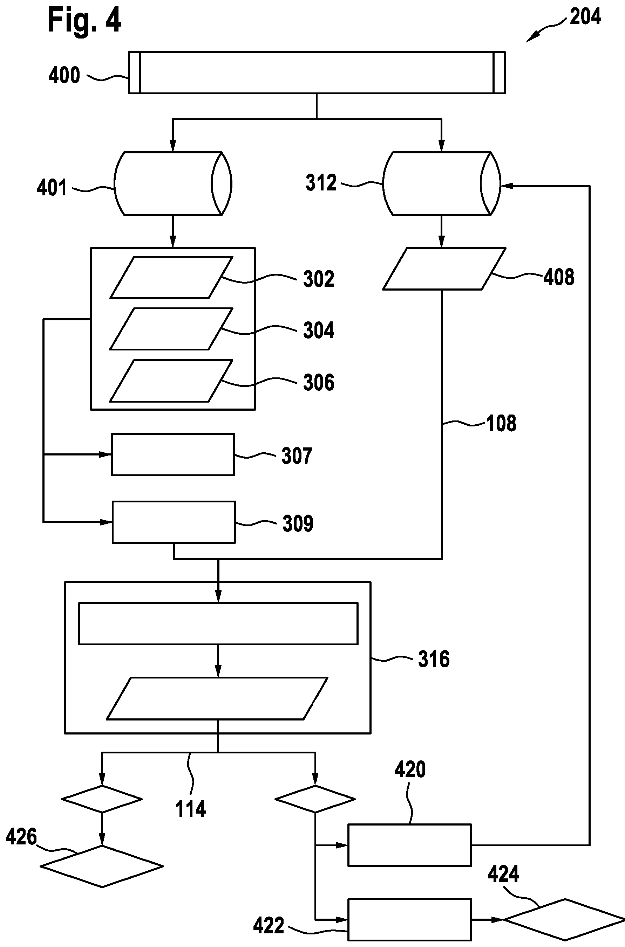

Fig. 2 zeigt einen schematischen Ablauf eines Verfahrens 200 zur Qualitätskontrolle eines Atemmessgeräts gemäß einem Ausführungsbeispiel mit einer Auswahlmöglichkeit 201 zwischen zwei Teilverfahren 202, 204. Das Verfahren 200 ist in einer Vorrichtung anwendbar, wie sie in Fig. 1 beschrieben wurde. Gemäß diesem Ausführungsbeispiel umfasst das Verfahren 200 zur Qualitätskontrolle des Atemmessgeräts das erste Teilverfahren 202 zum Qualifizieren einer Testperson, wie es nachfolgend anhand von Fig. 3 näher beschrieben wird, und das zweite Teilverfahren 204 zur Qualitätsprüfung des Atemmessgeräts, wie es nachfolgend anhand von Fig. 4 näher beschrieben wird. Somit lässt sich eine zweiteilige Qualitätskontrolle realisieren.

Die Teilverfahren 202, 204 können zeitlich nacheinander ausgeführt werden. Dabei kann zunächst im ersten Teilverfahren 202 überprüft werden, ob sich eine Person als Testperson qualifiziert. Wenn dies der Fall ist, können basierend auf Atemproben dieser Person gebildete Referenzwerte für eine nachfolgende Durchführung des zweiten Teilverfahrens 204 gespeichert oder bereitgestellt werden. In dem zweiten Teilverfahren 204 können die Referenzwerte verwendet werden, um die die Qualitätsprüfung des Atemmessgeräts durchzuführen. Das zweite Teilverfahren 204 wird beispielsweise in vorbestimmten Abständen wiederholt ausgeführt, um basierend auf den einmal gebildeten Referenzwerten die Qualitätsprüfung durchzuführen. Durch die Qualitätsprüfung wird beispielsweise festgestellt, ob das Atemmessgerät korrekt funktioniert oder aber defekt ist. Die Teilverfahren 202, 204 können auch unabhängig voneinander ausgeführt werden oder es kann nur eines der Teilverfahren 202, 204 ausgeführt werden.

Das Verfahren 200 ermöglicht die Qualitätskontrolle unter Verwendung erfasster Messwerte des Nutzers und unter Berücksichtigung einer Korrelation zwischen einem Niveau, beispielsweise einem Mittelwert und der Standardabweichung dieser erfassten Messwerte. Zum Erfassen eines Messwerts kann der Nutzer aufgefordert werden, um in das Atemmessgerät auszuatmen, um die Atemluft des Nutzers unter Verwendung des Atemmessgeräts analysieren zu können und ein Ergebnis der Analyse als den Messwert bereitstellen zu können.

Bei einem geringen Niveau der NO-Konzentration in der Ausatemluft, kurz "FeNO-Niveau", ist auch die natürliche Schwankung der Messwerte und damit die Standardabweichung "SD" geringer als bei einem hohen Niveau. Diese Tatsache wird nun ausgenutzt, um die Breite eines Toleranzbands sowohl für die Eignung des Nutzers als Testperson als auch für die Gültigkeit der Qualitätssicherung festzulegen.

Dabei fällt die Qualitätskontrolle positiv aus, wenn eine Testmessung innerhalb des Toleranzbandes fällt, wobei sich die Breite des Toleranzbandes unter Berücksichtigung der Gerätegenauigkeit G (beispielsweise +/- 5 ppb), des FeNO-Niveaus der Testperson und der Schwankung bisher erfasster Messwerte der Testperson, vorzugsweise als Gewichtung der SD, ergibt. Wenn das erste Teilverfahren 202 durchgeführt wird, bedeutet eine positive Qualitätskontrolle, dass die Person als Testperson qualifiziert ist und die Messwerte als Referenzwerte gespeichert werden. Wenn das zweite Teilverfahren 204 durchgeführt wird, bedeutet eine positive Qualitätskontrolle, dass das Atemmessgerät als funktionstüchtig kategorisiert wird. Die Ergebnisse beider Teilverfahren 202, 204, also das Ergebnis des Qualifizierens der Testperson als auch das Ergebnis der Qualitätsprüfung des Atemmessgeräts, werden gemäß einem Ausführungsbeispiel in einem Gerätespeicher des Atemmessgeräts abgelegt.

Gemäß einem Ausführungsbeispiel antikorrelieren das FeNO-Niveau und die Schwankung (insbesondere in Form der Standardabweichung) in der Bestimmung der Breite des Toleranzbandes.

Dazu wird gemäß einem Ausführungsbeispiel ein Schwellenwert (bspw. 25 ppb) für einen FeNO-Wert vorgegeben, unterhalb dessen die Schwankung mit einem ersten Gewichtswert in die Breite des Toleranzbandes eingeht. Der Gewichtswert, auch als Faktor bezeichnet, beträgt beispielsweise "3". Oberhalb des vorgegebenen FeNO-Schwellenwerts geht die Schwankung mit einem zweiten Gewichtswert, beispielsweise "2" ein. Dies bedeutet also beispielsweise: Bandbreite: $(MW - G - 3*SD) \leq x4 \leq (MW + G + 3*SD)$ für FeNO < 25 ppb und $(MW - G - 2*SD) \leq x4 \leq (MW + G + 2*SD)$ für FeNO >= 25 ppb, wobei x4 der aktuell gemessene (und zu überprüfende) FeNO-Wert der Testperson und G die Gerätegenauigkeit bezeichnen. "FeNO" bezeichnet dabei insbesondere einen vorher gemessenen FeNO-Wert der Testperson oder einen Mittelwert von mehreren vorher gemessenen FeNO-Werten der Testperson, beispielsweise einen Mittelwert von drei vorher gemessenen FeNO-Werten der Testperson.

Gemäß einem Ausführungsbeispiel werden mehrere solcher FeNO-Werte mit jeweils verschiedenen Gewichtswerten für die Schwankung vorgegeben.

Anstelle des vorgegebenen FeNO-Werts kann eine vorgegebene Standardabweichung zum Festlegen des Gewichtswerts verwendet werden, wie es beispielsweise anhand der Figuren 3 und 4 beschrieben ist.

Gemäß einem Ausführungsbeispiel wird der Nutzer aufgefordert, die Qualitätskontrolle, insbesondere das zweite Teilverfahren 204 nach einer Mindestdauer zu wiederholen, wenn die Qualitätskontrolle negativ ausfällt, die Testmessung also außerhalb des Toleranzbands liegt.

Dabei wird das Atemmessgerät gemäß einem Ausführungsbeispiel, zumindest für eine Mindestdauer, gesperrt, wenn die Qualitätskontrolle negativ ausfällt. Fig. 3 zeigt ein Ablaufdiagramm eines Verfahrens 202 zum Qualifizieren einer Testperson gemäß einem Ausführungsbeispiel. Das Verfahren 202 entspricht dem in Fig. 2 genannten ersten Teilverfahren zum Qualifizieren einer Testperson und kann beispielsweise in einem Atemmessgerät durchgeführt werden, wie es in Fig. 1 beschrieben wurde. Gemäß diesem Ausführungsbeispiel werden im Verfahren 202 nach einem Start 300 des Verfahrens 202 in einem Schritt 301 des Erfassens ein erster Referenzwert 302, ein zweiter Referenzwert 304 und ein dritter Referenzwert 306 erfasst, aus denen beispielsweise in einem Schritt 307 ein Mittelwert und in einem Schritt 309 eine Standardabweichung bestimmt werden. Bei den Referenzwerten 302, 304, 306 handelt es sich beispielsweise um von der in Fig. 1 gezeigten Analyseeinrichtung bereitgestellte Messwerte, die einer Person zugeordnet sind. Das Verfahren 202 wird ausgeführt, um zu überprüfen, ob diese Person als Testperson qualifiziert ist und die Referenzwerte 302, 304, 306 zur Qualitätsprüfung des Atemmessgeräts geeignet sind.

Gemäß diesem Ausführungsbeispiel wird der Mittelwert (MW) im Schritt 307 nach der folgenden Formel bestimmt:

$$MW = \frac{\sum_{i=1}^{n} x_i}{n}$$

Die Standardabweichung (SD) wird dem entsprechend im Schritt 309 nach der folgenden Formel bestimmt:

$$SD = \sqrt{\frac{1}{n-1} \sum_{i=1}^{n} (x_i - \overline{x})^2}$$

Gemäß diesem Ausführungsbeispiel umfasst das Verfahren 202 weiterhin einen Schritt 312 des Einlesens. Im Schritt 312 des Einlesens wird über eine interne Schnittstelle ein Prüfwert 308 eingelesen, der eine unter Verwendung des Atemmessgeräts gemessene Gaskonzentration repräsentiert. Bei dem Prüfwert 308 handelt es sich beispielsweise um einen von der in Fig. 1 gezeigten Analyseeinrichtung bereitgestellten Messwert, der derselben Person zugeordnet ist, wie die Referenzwerte 302, 304, 306. In einem Schritt 314 des Bildens wird gemäß der Erfindung ein erster Schwellenwert und ein zweiter Schwellenwert gebildet, die unter Verwendung des Mittelwerts und der Standardabweichung der Referenzwerte 302, 304, 306 gebildete Werte repräsentieren. Gemäß diesem Ausführungsbeispiel wird die Standardabweichung zum Bilden der Schwellenwerte mit einem ersten Faktor multipliziert, wenn die Standardabweichung kleiner ist als ein vorbestimmter Gaskonzentrationswert, und mit einem zweiten Faktor multipliziert, wenn die Standardabweichung größer ist als der vorbestimmte Gaskonzentrationswert.

In einem Schritt 316 des Vergleichens wird daraufhin der Prüfwert 308 gemäß diesem Ausführungsbeispiel mit dem ersten Schwellenwert und dem zweiten Schwellenwert verglichen, um das Vergleichsergebnis 114 zu erhalten.

Ist der Prüfwert 308 größer als der erste Schwellenwert und kleiner als der zweite Schwellenwert, so werden optional in einem Schritt 318 des Speicherns die Referenzwerte 302, 204, 306 für eine nachfolgende Qualitätsprüfung gespeichert und in einem optionalen Schritt 320 des Ausgebens ein Qualitätsignal 322 ausgegeben, das eine bestandene Qualifizierung der Testperson anzeigt. Nachfolgend kann das anhand von Fig. 4 beschriebene Verfahren ausgeführt werden, bei dem die Referenzwerte 302, 204, 306 zur Qualitätsprüfung des Atemmessgeräts verwendet werden.

Ist dagegen der Prüfwert 308 kleiner als der erste Schwellenwert oder größer als der zweite Schwellenwert, so wird gemäß einem Ausführungsbeispiel in einem Schritt 324 eine Auswahl einer anderen Testperson angezeigt. In diesem Fall wird das Verfahren 202 beginnend mit dem Start 300 erneut ausgeführt. Es werden also erneut die Schritte 301, 312 ausgeführt um weitere Referenzwerte und einen weiteren Prüfwert einzulesen, die einer weiteren Person zugeordnet sind. Die weiteren Referenzwerte und der weitere Prüfwert werden in entsprechender Weise durch erneutes Ausführen der Schritte 307, 309, 314, 316 verwendet, um zu überprüfen, ob sich die weitere Person als Testperson qualifiziert. Wenn dies der Fall ist, so können die weiteren Referenzwerte für eine nachfolgende Qualitätsprüfung des Atemmessgeräts verwendet werden.

Das Verfahren 202 kann solange wiederholt ausgeführt werden, bis eine Qualifizierung einer Person als Testperson erfolgt ist.

Mit anderen Worten wird durch das Verfahren 200 ermöglicht, eine Qualitätssicherung mit menschlichen Proben zu verbessern, indem physiologische Schwankungen erkannt und nicht als Fehler im Atemmessgerät klassifiziert werden.

Darüber hinaus werden Unterschiede zwischen Testpersonen besser berücksichtigt, womit die Probe für die Qualitätssicherung zuverlässiger bereitgestellt werden kann.

Gemäß diesem Ausführungsbeispiel ist es für das Verfahren 202 vorteilhaft, wenn Messungen innerhalb einer Woche durchgeführt werden, um die Referenzwerte 302, 304, 306 zu erhalten, wobei pro Tag nur eine Messung durchgeführt wird. Gemäß diesem Ausführungsbeispiel handelt es sich bei den drei Referenzwerten 302, 304, 306 um FeNO-Werte, die auch als FeNO-Werte $[x_1 \ldots x_3]$ bezeichnet werden und durch drei FeNO-Messungen bestimmt werden. Anstelle der drei Referenzwerte 302, 304, 306 kann in entsprechender Weise auch eine andere Anzahl von Referenzwerten verwendet werden.

Nach der Durchführung der drei Messungen zum Bestimmen der Referenzwerte 302, 304, 306 folgt eine Berechnung des Mittelwerts und der Standardabweichung aus den beispielhaft drei Referenzwerten 302, 304, 306, beispielsweis wie es anhand der Schritte 307, 309 beschrieben ist.

Aus der Durchführung einer weiteren, hier einer vierten Messung ergibt sich der Prüfwert 308. Gemäß diesem Ausführungsbeispiel handelt es sich bei dem Prüfwert 308 um einen weiteren FeNO-Wert, der auch als FeNO-Wert $x_4$ bezeichnet wird und durch eine weitere FeNO-Messung bestimmt wird.

Die Person, deren Atemproben zum Bestimmen der Referenzwerte 302, 304, 306 und des Prüfwerts 308 verwendet wurden, gilt vorzugsweise als Testperson geeignet, wenn die folgenden Bedingungen erfüllt sind: Vorzugsweise FeNO-Werte $[x_1 \ldots x_4] < 50$ ppb, und

$$(MW - G - 3*SD) \leq x4 \leq (MW+G+3*SD) \text{ für } SD < 2{,}5 \text{ ppb}$$

oder

$$(MW - G - 2*SD) \leq x4 \leq (MW+G+2*SD) \text{ für } SD > 2{,}5 \text{ ppb.}$$

Dabei steht G für die Genauigkeit des Atemmessgeräts, wobei eine Genauigkeit des Atemmessgeräts im Messbereich FeNO als kleiner 50ppb angenommen wird und beispielhaft bei 5ppb liegt. Wie ausgeführt beträgt der erste Faktor beispielsweise 3, für den Fall, dass die Standardabweichung kleiner als der vorbestimmte Gaskonzentrationswert von hier beispielsweise 2,5 ppb ist, Und der zweite Faktor beträgt beispielsweise 2, für den Fall, das die Standardabweichung größer als der vorbestimmte Gaskonzentrationswert ist. Wie anhand von Fig. 2 ausgeführt, kann auch ein vorbestimmter Gaskonzentrationswert für den FeNO-Wert anstelle der Standardabweichung verwendet werden, um den jeweiligen Faktor zu definieren, also vorzugsweise, wenn wenn die folgenden Bedingungen erfüllt sind:

$$(MW - G - 3*SD) \leq x4 \leq (MW+G+3*SD) \text{ für } FeNO < 25ppb$$

oder

$$(MW - G - 2*SD) \leq x4 \leq (MW+G+2*SD) \text{ für } FeNO >= 25ppb.$$

Auch können eine Mehrzahl von Gaskonzentrationswerten oder Gaskonzentrationswertbereichen vorbestimmt sein, um eine Mehrzahl von Faktoren vorzugeben. Gemäß einem Ausführungsbeispiel geht die Genauigkeit des Atemmessgeräts durch eine einfache Addition und/oder Subtraktion in die Bestimmung der Schwellenwerte ein. Beispielsweise wird die Genauigkeit zur Bestimmung des ersten Schwellenwerts von dem Mittelwert subtrahiert und zur Bestimmung des zweiten Schwellenwerts zu dem Mittelwert addiert.

Fig. 4 zeigt ein Ablaufdiagramm eines Verfahrens 204 zur Qualitätsprüfung eines Atemmessgeräts gemäß einem Ausführungsbeispiel. Das Verfahren 204 wird gemäß diesem Ausführungsbeispiel in einem Atemmessgerät durchgeführt, wie es in Fig. 1 beschrieben wurde. Das Verfahren 204 ist ebenfalls als Teilverfahren durchführbar, wie es in Fig. 2 beschrieben wurde.

Gemäß diesem Ausführungsbeispiel umfasst das Verfahren 204 nach einem Start 400 des Verfahrens 204 einen Schritt 401 des Einlesens von Referenzwerten 302, 304, 306. Bei den Referenzwerten 302, 304, 306 handelt es sich um die im anhand von Fig. 3 beschriebenen Referenzwerte, sowie optional den anhand von Fig. 3 beschriebenen Prüfwert und, sofern das Verfahren 204 bereits ausgeführt wurde, ferner um gespeicherte Messwerte aus einer bereits durchgeführten Qualitätsprüfung des Atemmessgeräts. Die Referenzwerte 302, 304, 306 werden beispielsweise über eine interne Schnittstelle zu einer Speichereinrichtung eingelesen, in der die Referenzwerte 302, 304, 306 gespeichert sind. Basierend auf den Referenzwerten 302, 304, 306 erfolgt in einem Schritt 307 eine Berechnung des Mittelwerts und in einem Schritt 309 eine Berechnung der Standardabweichung, wie es bereits anhand von Fig. 3 beschrieben wurde.

In einem Schritt 312 wird ein Prüfwert 408 eingelesen, der eine unter Verwendung des Atemmessgeräts gemessene Gaskonzentration repräsentiert.

Der Prüfwert wird beispielsweise über eine interne Schnittstelle zu einer Analyseeinrichtung eingelesen, durch die der Prüfwert 408 generiert wurde. Bei dem Prüfwert 408 handelt es sich somit beispielsweise um einen von der in Fig. 1 gezeigten Analyseeinrichtung bereitgestellten Messwert.

Gemäß diesem Ausführungsbeispiel wird in einem Schritt 316 des Vergleichens der im Schritt 312 des Einlesens eingelesene Prüfwert 408 mit einem ersten Schwellenwert und einem zweiten Schwellenwert verglichen, um ein Vergleichsergebnis 114 zu erhalten. Dabei repräsentieren der erste Schwellenwert und der zweite Schwellenwert unter Verwendung des Mittelwerts und der Standardabweichung gebildete Werte. Die Bildung der Schwellenwerte sowie die Durchführung des Vergleichs im Schritt 312 erfolgt dabei wie bereits anhand von Fig. 3 beschrieben.

Zeigt das Vergleichsergebnis 114 aus dem Schritt 316 an, dass der Prüfwert 408 kleiner ist als der erste Schwellenwert oder größer ist als der zweite Schwellenwert, dann kann dies daran liegen, dass der Prüfwert 408 nicht geeignet war oder das Atemmessgerät defekt ist. Sofern das Verfahren 204 bezüglich der aktuellen Qualitätsprüfung des Atemmessgeräts nur einmal ausgeführt wurde, der Prüfwert 408 also einer ersten Messung entstammt, so wird die Messung nach einer Wartezeit, von beispielsweise fünf Minuten erneut durchgeführt. Dies ist durch die Verzweigung 420 angedeutet. In diesem Fall werden die Schritte 312, 316 erneut ausgeführt, um einen weiteren Prüfwert einzulesen und mit den Schwellenwerten zu vergleichen. Wenn das Verfahren 204 bezüglich der aktuellen Qualitätsprüfung des Atemmessgeräts bereits zweimal ausgeführt wurde, der weitere Prüfwert also bereits aus einer zweiten Messung entstammt, so wird das Atemmessgerät in einem Schritt 424 als defekt deklariert, wenn die Testperson gesund ist. Dies ist durch die Verzweigung 422 angedeutet. In diesem Fall wird das Verfahren 204 beendet.

Um zu überprüfen, ob die Testperson gesund ist, umfasst das Verfahren 204 optional einen Schritt des Bereitstellens eines Anzeigesignals an eine Anzeigeeinrichtung des Atemmessgeräts. Das Anzeigesignal ist geeignet, um eine an die Testperson gerichtete Frage bezüglich des Gesundheitszustands der Testperson zu richten. Die Frage kann beispielsweise über eine Anzeigeeinrichtung, beispielsweise über ein Display, angezeigt werden. Negiert der Nutzer die Frage beispielsweise via Eingabe in das Atemmessgerät, so wird das Atemmessgerät im Schritt 424 als defekt kategorisiert.

Zeigt dagegen das Vergleichsergebnis 114 aus dem Schritt 316 an, dass der Prüfwert 408 größer als der erste Schwellenwert und kleiner als der zweite Schwellenwert ist, dann wird das Atemmessgerät in einem Schritt 426 als funktionstüchtig kategorisiert. In anderen Worten hat das Atemmessgerät in einem solchen Fall die Qualitätsprüfung bestanden.

Mit anderen Worten sind gemäß diesem Ausführungsbeispiel die Referenzwerte 302, 304, 306 im Atemmessgerät gespeichert, sodass aus ihnen der Mittelwert und die Standardabweichung berechnet werden kann. Neben den Referenzwerte 302, 304, 306 sowie optional dem Prüfwert aus der anhand von Fig. 3 beschriebenen Qualifizierung der Testperson werden auch aus bisher durchgeführten Messungen im Rahmen der Qualitätsprüfung hervorgegangene Messwerte als Referenzwerte verwendet.

Der Mittelwert und die Standardabweichung können wie bereits beschrieben nach den folgenden Formeln bestimmt werden:

$$MW = \frac{\sum_{i=1}^{n} x_i}{n}$$

$$SD = \sqrt{\frac{1}{n-1} \sum_{i=1}^{n} (x_i - \overline{x})^2}$$

Zur Überprüfung der Gerätequalität führt der Nutzer eine Messung $x_{n+1}$ durch, wobei der daraus resultierende Prüfwert 408 mit den bisher durchgeführten Qualitätsmessungen, beispielsweise mit den Referenzwerten 302, 304, 306 verglichen wird.

Die Messung und damit die Qualitätssicherung sind gültig, wenn

$$(MW - G - 3*SD) \leq x_{n+1} \leq (MW + G + 3*SD) \text{ für } SD < 2,5 \text{ ppb}$$

oder

$$(MW - G - 2*SD) \leq x_{n+1} \leq (MW + G + 2*SD) \text{ für } SD > 2,5 \text{ ppb}.$$

gegeben ist. Dabei gelten die weiteren im Zusammenhang mit der Fig. 3 erläuterten Ausführungen, also insbesondere alternativ die oben genannten Bedingungen für eine gültige Messung und damit Qualitätssicherung:

$$(MW - G - 3*SD) \leq x_{n+1} \leq (MW + G + 3*SD) \text{ für FeNO} < 25\text{ppb}$$

oder

$$(MW - G - 2*SD) \leq x_{n+1} \leq (MW + G + 2*SD) \text{ für FeNO} \geq 25\text{ppb}.$$

Liegt der Prüfwert 408 außerhalb dieses Schwellenbereichs, wird der Nutzer veranlasst, erneut eine Messung durchzuführen, also beispielsweise die Messung aus der der Prüfwert 408 resultiert, nach 5 Minuten zu wiederholen. Liegt der Prüfwert 408 danach innerhalb des Schwellenbereichs, gilt die Qualitätsprüfung als bestanden. Der vorangegangene Messwert wird als Ausreißer angesehen und verworfen.

Liegt der Prüfwert 108 jedoch erneut außerhalb des Schwellenbereichs, so wird das Gerät bei dem Nutzer erfragen, ob beispielsweise eine Erkältung oder eine Krankheit vorliegt. Ist dies der Fall, muss die Qualitätsprüfung mit einem anderen Nutzer wiederholt werden. Kann ein Infekt oder Ähnliches jedoch ausgeschlossen werden, wird der Prüfwert 408 oder der weitere Prüfwert der erneuten Messung in die Betrachtung eingeschlossen und die Qualitätsprüfung gilt als nicht bestanden. In diesem Fall ist von einem Gerätefehler auszugehen und es können entsprechende Maßnahmen mit einem technischen Dienst des Herstellers eingeleitet werden.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

**Patentansprüche**

1. Verfahren (200) zur Qualitätskontrolle eines Atemmessgeräts (100), wobei das Verfahren (200) ein erstes Teilverfahren (202) zum Qualifizieren einer Testperson und/oder ein zweites Teilverfahren (204) zur Qualitätsprüfung des Atemmessgeräts (100) mit den folgenden Schritten umfasst:

   Bestimmen eines Mittelwerts und einer Schwankung unter Verwendung von Referenzwerten (302, 304, 306), die mit dem Atemmessgerät (100) gemessene Gaskonzentrationen repräsentieren;
   Vergleichen (316) eines Prüfwerts (108; 308; 408) mit einem Toleranzband, wobei eine Breite des Toleranzbands durch einen ersten Schwellenwert und einen zweiten Schwellenwert begrenzt wird, wobei der erste Schwellenwert und der zweite Schwellenwert unter Verwendung des Mittelwerts und der Schwankung von Referenzwerten (302, 304, 306) sowie einer Genauigkeit des Atemmessgeräts (100) gebildete Werte repräsentieren, wobei der Prüfwert (108; 308; 408) mit dem Atemmessgerät (100) gemessene Gaskonzentrationen repräsentieren; und durch folgende Schritte gekennzeichnet:
   Speichern (318) der Referenzwerte (302, 304, 306) für eine nachfolgende Durchführung des zweiten Teilverfahrens (204) wenn der Prüfwert (108; 308) innerhalb des Toleranzbands liegt und das erste Teilverfahren (202) zum Qualifizieren einer Testperson durchgeführt wird, oder Kategorisieren (426) des Atemmessgeräts (100) als funktionstüchtig, wenn der Prüfwert (108; 308) innerhalb des Toleranzbands liegt und das zweite Teilverfahren (204) zur Qualitätsprüfung des Atemmessgeräts (100) durchgeführt wird.

2. Verfahren (200, 202, 204) gemäß Anspruch 1, mit einem Schritt (314) des Bildens des ersten Schwellenwerts und des zweiten Schwellenwerts unter Verwendung des Mittelwerts und der Schwankung und der Genauigkeit des Atemmessgeräts (100).

3. Verfahren gemäß Anspruch 2, bei dem im Schritt (314) des Bildens durch den ersten Schwellenwert und den zweiten Schwellenwert eine Bestimmung der Breite des Toleranzbands erfolgt, wobei der Mittelwert und die Schwankung in der Bestimmung der Breite antikorrelieren.

4. Verfahren (200, 202, 204) gemäß Anspruch 2 oder 3, wobei im Schritt (314) des Bildens die Schwankung in Form der Standardabweichung der Referenzwerte (302, 304, 306) zum Bilden der Schwellenwerte mit einem ersten Faktor multipliziert wird, wenn die Standardabweichung oder der Mittelwert kleiner ist als ein vorbestimmter Gaskonzentrationswert ist, und mit einem zweite Faktor multipliziert wird, wenn die Standardabweichung oder der Mittelwert größer ist als der vorbestimmte Gaskonzentrationswert.

5. Verfahren (200, 202, 204) gemäß einem der Ansprüche 2 bis 4, wobei im Schritt (314) des Bildens die Schwankung in Form der Standardabweichung der Referenzwerte (302, 304, 306) zum Bilden der Schwellenwerte mit einem von dem Mittelwert abhängigen Faktor multipliziert wird.

6. Verfahren (200, 202) gemäß einem der vorangegangenen Ansprüche, mit einem Schritt (301) des Erfassens der Referenzwerte (302, 304, 306) unter Verwendung des Atemmessgeräts (100).

7. Verfahren (200 204) gemäß einem der vorangegangenen Ansprüche, mit einem Schritt (312) des Einlesens des Prüfwerts, wobei der Schritt (312) nach einer Mindestdauer erneut ausgeführt wird, um zum Veranlassen einer Wiederholung des zweiten Teilverfahrens (204) einen weiteren Prüfwert einzulesen, der eine unter Verwendung des Atemmessgeräts (100) gemessene weitere Gaskonzentration repräsentiert, wenn der Prüfwert (108; 308; 408) außerhalb des Toleranzbands liegt.

8. Verfahren (200, 204) gemäß Anspruch 7, mit einem Schritt des Bereitstellens eines Anzeigesignals, das eine an einen Nutzer gerichtete Frage repräsentiert, wenn nach dem erneuten Ausführen des Schritts (312) der weitere Prüfwert (108; 408) kleiner ist als der erste Schwellenwert oder größer ist als der zweite Schwellenwert.

9. Verfahren (204) gemäß einem der vorangegangenen Ansprüche, bei dem ein Schritt (312) des Einlesens eines weiteren Prüfwerts unterbunden wird, wenn der Prüfwert (408) außerhalb des Toleranzbands liegt.

10. Verfahren (204) gemäß einem der vorangegangenen Ansprüche, bei dem zuerst das erste Teilverfahren (202) zum Qualifizieren einer Testperson und anschließend das zweites Teilverfahren (204) zur Qualitätsprüfung des Atemmessgeräts (100) durchgeführt wird.

11. Vorrichtung (102), die eingerichtet ist, um die Schritte des Verfahrens (200, 202, 204) gemäß einem der vorangegangenen Ansprüche in einer Recheneinheit und einer Speichereinheit, insbesondere einer Einleseeinheit (110) und einer Vergleichseinheit (112) auszuführen und/oder anzusteuern.

12. Atemmessgerät (100) mit einer Vorrichtung (102) gemäß Anspruch 11, wobei das Atemmessgerät (100) ausgebildet ist um eine Gaskonzentration zu messen.

13. Computerprogramm, umfassend Befehle, die bewirken, dass die Vorrichtungen der Ansprüche 11 oder 12 die Verfahrensschritte nach den Ansprüchen 1 bis 10 ausführt.

14. Maschinenlesbares Speichermedium, auf dem das Computerprogramm nach Anspruch 13 gespeichert ist.

**Claims**

1. Method (200) for quality control of a breath measuring device (100), wherein the method (200) comprises a first sub-method (202) for qualifying a test subject and/or a second sub-method (204) for testing the quality of the breath measuring device (100), comprising the following steps:

   determining an average value and a fluctuation using reference values (302, 304, 306) representing gas concentrations measured with the breath measuring device (100);
   comparing (316) a test value (108; 308; 408) with a tolerance band, wherein a width of the tolerance band is limited by a first threshold value and a second threshold value, wherein the first threshold value and the second threshold value represent values formed using the average value and the fluctuation of reference values (302, 304, 306) as well as an accuracy of the breath measuring device (100), wherein the test value (108; 308; 408) represents gas concentrations measured with the breath measuring device (100); and
   **characterized by** the following steps:
   storing (318) the reference values (302, 304, 306) for subsequently performing the second sub-method (204) if the test value (108; 308) lies within the tolerance band and the first sub-method (202) for qualifying a test subject is performed, or categorizing (426) the breath measuring device (100) as functional if the test value (108; 308) lies within the tolerance band and the second sub-method (204) for testing the quality of the breath measuring device (100) is performed.

2. Method (200, 202, 204) according to Claim 1, comprising a step (314) of forming the first threshold value and the second threshold value using the average value and the fluctuation and the accuracy of the breath measuring device (100).

3. Method according to Claim 2, in which the width of the tolerance band is determined in the forming step (314) by means of the first threshold value and the second threshold value, wherein the average value and the fluctuation anti-correlate in the determination of the width.

4. Method (200, 202, 204) according to Claim 2 or 3, wherein, in the forming step (314), the fluctuation in the form of the standard deviation of the reference values (302, 304, 306) for forming the threshold values is multiplied by a first factor if the standard deviation or the average value is less than a predetermined gas concentration value, and is multiplied by a second factor if the standard deviation or the average value is greater than the predetermined gas concentration value.

5. Method (200, 202, 204) according to one of Claims 2 to 4, wherein, in the forming step (314), the fluctuation in the form of the standard deviation of the reference values (302, 304, 306) for forming the threshold values is multiplied by a factor dependent on the average value.

6. Method (200, 202) according to one of the preceding claims, comprising a step (301) of capturing the reference values (302, 304, 306) using the breath measuring device (100).

7. Method (200, 204) according to one of the preceding claims, comprising a step (312) of reading in the test value, wherein the step (312) is carried out again after a minimum period in order, for the purpose of prompting repetition of the second sub-method (204), to read in a further test value representing a further gas concentration measured using the breath measuring device (100) if the test value (108; 308; 408) lies outside the tolerance band.

8. Method (200, 204) according to Claim 7, comprising a step of providing a display signal representing a question directed to a user if, after the step (312) has been carried out again, the further test value (108; 408) is less than the first threshold value or greater than the second threshold value.

9. Method (204) according to one of the preceding claims, in which a step (312) of reading in a further test value is prevented if the test value (408) lies outside the tolerance band.

10. Method (204) according to one of the preceding claims, in which first the first sub-method (202) for qualifying a test subject and then the second sub-method (204) for testing the quality of the breath measuring device (100) are carried out.

11. Apparatus (102) configured to carry out and/or control the steps of the method (200, 202, 204) according to one of the preceding claims in a computing unit and a storage unit, in particular a read-in unit (110) and a comparison unit (112).

12. Breath measuring device (100) having an apparatus (102) according to Claim 11, wherein the breath measuring device (100) is designed to measure a gas concentration.

13. Computer program, comprising instructions that cause the apparatus in Claim 11 to 12 to carry out the method steps according to Claims 1 to 10.

14. Machine-readable storage medium on which the computer program according to Claim 13 is stored.


**Revendications**

1. Procédé (200) de contrôle de qualité d'un appareil de mesure respiratoire (100), le procédé (200) comprenant un premier sous-procédé (202) de qualification d'une personne test et/ou un deuxième sous-procédé (204) de contrôle de qualité de l'appareil de mesure respiratoire (100), comprenant les étapes suivantes :

   détermination d'une valeur moyenne et d'une fluctuation en utilisant des valeurs de référence (302, 304, 306) représentant des concentrations de gaz mesurées avec l'appareil de mesure respiratoire (100) ;
   comparaison (316) d'une valeur de contrôle (108 ; 308 ; 408) à une bande de tolérance, une largeur de la bande de tolérance étant limitée par une première valeur de seuil et une deuxième valeur de seuil, la première valeur de seuil et la deuxième valeur de seuil étant formées en utilisant la valeur moyenne et la fluctuation de valeurs de référence (302, 304, 306) ainsi qu'une précision de l'appareil de mesure respiratoire (100), la valeur de contrôle (108 ; 308 ; 408) représentant des concentrations de gaz mesurées avec l'appareil de mesure respiratoire (100) ; et

**caractérisé par** les étapes suivantes :

stockage (318) des valeurs de référence (302, 304, 306) pour une exécution ultérieure du deuxième sous-procédé (204) si la valeur de contrôle (108 ; 308) se situe à l'intérieur de la bande de tolérance et si le premier sous-procédé (202) est exécuté pour qualifier une personne test, ou catégorisation (426) de l'appareil de mesure respiratoire (100) comme étant fonctionnel si la valeur de contrôle (108 ; 308) se situe à l'intérieur de la bande de tolérance et si le deuxième sous-procédé (204) de contrôle de qualité de l'appareil de mesure respiratoire (100) est exécuté.

2. Procédé (200, 202, 204) selon la revendication 1, comprenant une étape (314) de formation de la première valeur de seuil et de la deuxième valeur de seuil en utilisant la valeur moyenne, la fluctuation et la précision de l'appareil de mesure respiratoire (100).

3. Procédé selon la revendication 2, dans lequel, lors de l'étape (314) de formation par la première valeur de seuil et la deuxième valeur de seuil, une détermination de la largeur de la bande de tolérance est effectuée, la valeur moyenne et la fluctuation étant anti-corrélées lors de la détermination de la largeur.

4. Procédé (200, 202, 204) selon la revendication 2 ou 3, dans lequel, lors de l'étape (314) de formation, la fluctuation sous la forme de l'écart-type des valeurs de référence (302, 304, 306) pour former les valeurs de seuil est multipliée par un premier facteur si l'écart-type ou la valeur moyenne est inférieur à une valeur de concentration de gaz prédéterminée, et est multipliée par un deuxième facteur si l'écart-type ou la moyenne est supérieur à la valeur de concentration de gaz prédéterminée.

5. Procédé (200, 202, 204) selon l'une des revendications 2 à 4, dans lequel, lors de l'étape (314) de formation, la fluctuation sous la forme de l'écart type des valeurs de référence (302, 304, 306) pour former les valeurs de seuil est multipliée par un facteur dépendant de la valeur moyenne.

6. Procédé (200, 202) selon l'une des revendications précédentes, comprenant une étape (301) d'acquisition des valeurs de référence (302, 304, 306) en utilisant l'appareil de mesure respiratoire (100).

7. Procédé (200 204) selon l'une des revendications précédentes, comprenant une étape (312) de lecture de la valeur de contrôle, dans lequel l'étape (312) est effectuée à nouveau après une durée minimale afin de provoquer une répétition du deuxième sous-procédé (204) pour lire une autre valeur de contrôle qui représente une autre concentration de gaz mesurée en utilisant l'appareil de mesure respiratoire (100), si la valeur de contrôle (108 ; 308 ; 408) se situe en dehors de la bande de tolérance.

8. Procédé (200, 204) selon la revendication 7, comprenant une étape de fourniture d'un signal d'affichage qui représente une question adressée à un utilisateur si, après l'exécution réitérée de l'étape (312), l'autre valeur de contrôle (108 ; 408) est inférieure à la première valeur de seuil ou supérieure à la deuxième valeur de seuil.

9. Procédé (204) selon l'une des revendications précédentes, dans lequel une étape (312) de lecture d'une autre valeur de contrôle est empêchée si la valeur de contrôle (408) se situe en dehors de la bande de tolérance.

10. Procédé (204) selon l'une des revendications précédentes, dans lequel le premier sous-procédé (202) de qualification d'une personne test est exécuté en premier, suivi du deuxième sous-procédé (204) de contrôle de qualité de l'appareil de mesure respiratoire (100).

11. Dispositif (102) conçu pour exécuter et/ou commander les étapes du procédé (200, 202, 204) selon l'une des revendications précédentes dans une unité de calcul et une unité de mémoire, en particulier une unité de lecture (110) et une unité de comparaison (112).

12. Appareil de mesure respiratoire (100) comportant un dispositif (102) selon la revendication 11, l'appareil de mesure respiratoire (100) étant conçu pour mesurer une concentration de gaz.

13. Programme informatique comprenant des instructions qui provoquent l'exécution des étapes de procédé selon les revendications 1 à 10 par les dispositifs selon les revendications 11 à 12.

**14.** Support de stockage lisible par machine sur lequel est stocké le programme informatique selon la revendication 13.

# Fig. 1

# Fig. 2

# Fig. 3

# Fig. 4

204

400

401

312

302

304

306

408

108

307

309

316

114

426

420

422

424

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2003216660 A1